# FASCICULE DE BREVET EUROPEEN

(11) **EP 0 932 375 B1**
(45) Date de publication et mention de la délivrance du brevet: **19.05.2004**
(21) Numéro de dépôt: 97931869.8
(22) Date de dépôt: 04.07.1997
(51) Int. Cl.: A61F 2/06

(54) **PROTHESE VASCULAIRE INTERNE EXPANSIBLE ET CINTRABLE A USAGE CHIRURGICAL**
FLEXIBLE UND EXPANDIERBARE, INTERNE, VASKULÄRE PROTHESE FÜR CHIRURGISCHE ANWENDUNG
FLEXIBLE AND EXPANSIBLE INTERNAL VASCULAR PROSTHESIS FOR SURGICAL USE

(43) Date de publication de la demande: 04.08.1999
(73) Titulaire: Fouere, Alain, 13008 Marseille (FR)
(72) Inventeur: Fouere, Alain, 13008 Marseille (FR)
(74) Mandataire: Roman, Michel
(86) Numéro de dépôt international: PCT/FR1997/001202
(87) Numéro de publication internationale: WO 1999/001088

(56) Documents cités:
- EP-A- 0 679 372
- EP-A- 0 686 379
- EP-A- 0 709 067
- DE-U- 29 702 671

## Description

La présente invention a pour objet la dilatation locale de conduits physiologiques tels que veines et artères dans le but de diminuer les troubles circulatoires et en particulier les risques d'infarctus provoqués par l'oblitération d'un vaisseau, par exemple du myocarde.
Ce type de prothèse est en soi connu et utilisé depuis plusieurs années. Il en existe plusieurs modèles, fabriqués en faisant appel à des techniques variées.
Par exemple, le brevet N° FR 2 728 156 déposé par l'auteur de la présente demande décrit une gaine chirurgicale constituée d'un élément tubulaire cylindrique dont la paroi forme un treillis ou grillage métallique malléable pouvant s'étendre radialement sous l'effet d'une pression interne et garder la forme ainsi obtenue, cet élément tubulaire comportant des moyens d'accrochage intégrés dirigés vers l'extérieur de façon à pouvoir pénétrer dans la paroi interne du vaisseau sous l'effet de la pression d'expansion, afin d'empêcher toute migration du dispositif.
Les dispositifs réalisés à ce jour présentent généralement l'inconvénient d'être rigides, ce qui rend leur utilisation délicate dans le cas de lésions situées dans une partie courbe du vaisseau à traiter ou s'étend sur une longueur importante. Dans ce dernier cas, il est généralement nécessaire de mettre en place plusieurs gaines extensibles à la suite les unes des autres, ce qui peut entraîner des interventions extrêmement lourdes.
Pour tenter de résoudre ce problème, la demande de brevet international numéro WO 98/16 172, du même inventeur, fait état d'une gaine extensible formée d'un tube composé d'au moins deux éléments non cintrables à structure en treillis expansible reliés l'un à l'autre par une zone cintrable formée de barres longitudinales en zigzag, l'ensemble étant agencé pour assurer une force radiale d'expansion contre la paroi interne du vaisseau d'un bout à l'autre de la gaine.
De même, le brevet N° EP 0 709 067 déposé par la société MEDINOL décrit une gaine vasculaire interne expansible constituée de plusieurs modules identiques non cintrables pouvant être reliés par des éléments flexibles en zigzag, ces modules étant formés de barres rectilignes repliées sur elles-mêmes pour former des lacets le long de la périphérie de la gaine.
Cependant, il subsiste toujours des éléments rigides de gaine et les barres en zigzag risquent, dans les parties comprimées, de créer des éléments proéminents susceptibles d'entraîner des lésions de la paroi interne du vaisseau si la gaine n'est pas mise en place avec suffisamment de précautions.

L'invention a pour but de remédier à ces inconvénients. En effet, elle permet de réaliser des gaines expansible destinées à assurer la dilatation de vaisseaux, ne comportant aucune partie rigide de manière à pouvoir s'adapter aux sinuosités du conduit dans lesquels elles sont placées.

Une gaine selon l'invention est constituée de modules cylindriques formés d'un assemblage de barres décrivant des méandres périphériques et comportant des éléments rectilignes longitudinaux sinueux susceptibles d'être allongés ou raccourcis, les barres étant solidarisées de manière à ce qu'un module soit composé de motifs fermés comprenant chacun quatre éléments rectilignes.

Sur les dessins annexés, donnés à titre d'exemples non limitatifs de formes de réalisation de l'objet de l'invention:
la figure 1 représente un module de gaine expansible sous forme développée,
la figure 2 montre à une échelle différente un motif fermé,
la figure 3 représente un fragment de module montrant la déformation des éléments rectilignes lors du cintrage de la gaine,
et les figures 4 et 5 sont deux exemples de gaines expansibles réalisées à partir de modules conformes à celui de la figure 1.

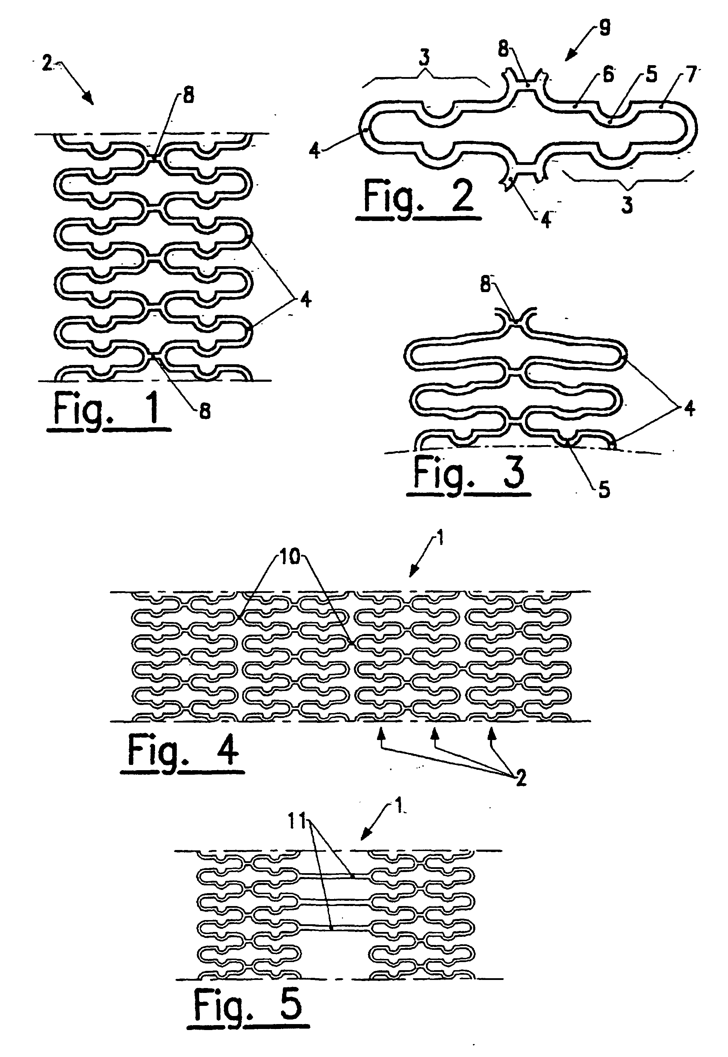

Le dispositif, figures 1 à 5, est constitué d'une gaine tubulaire 1 cylindrique de section circulaire, dont la paroi est ajourée de façon à former un grillage réalisé en matériau semi-rigide malléable agencé de manière à permettre à ladite gaine de s'étendre radialement sous l'effet d'une pression interne et de garder la forme ainsi obtenue, cette pression interne étant provoquée par exemple au moyen d'un ballonnet gonflable monté à l'extrémité d'un cathéter selon une technique connue.
La gaine 1 comporte un ou plusieurs modules 2 identiques constitués chacun de deux barres de section sensiblement constante parcourant la périphérie de la gaine en dessinant des méandres réguliers formés d'éléments longitudinaux 3 reliés par des éléments en arcs de cercles 4 alternés, la dimension de chaque méandre mesurée parallèlement à l'axe de la gaine 1 étant environ trois à quatre fois plus grande que la distance séparant deux éléments longitudinaux.
Ces derniers ne sont pas rectilignes mais formés d'une partie courbe 5 déformable insérée entre deux parties droites 6, 7 solidaires chacune d'un élément en arc de cercle 4.
Des barres courtes 8 relient les éléments en arc de cercle 4 respectifs de deux barres en méandres contiguës pour former un module 2, qui est ainsi constitué d'une succession périphérique de motifs fermés 9 (figure 2) comportant chacun quatre parties courbes 5.
Cette disposition permet au module 2, non seulement d'être extensible radialement, mais surtout de pouvoir être cintré pour épouser une partie courbe d'un conduit physiologique (figure 3). En outre, elle diminue la modification de la longueur de la gaine lors de la dilatation de celle-ci.

Une gaine 1 est constituée d'un nombre quelconque de modules 2 reliés entre eux par une ou plusieurs barres de liaison courtes 10, ou de barres longues 11 pouvant atteindre les deux tiers de la longueur d'un module, et disposées, selon les besoins, de façon contiguë (figure 5), en quinconce ou décalées périphériquement d'un module à l'autre (figure 4).

La gaine 1 est de préférence réalisée en une seule pièce par gravure ou usinage laser d'un tube métallique à paroi mince en acier inoxydable ou en tantale, ou par électro-érosion laser à partir d'un bloc de métal. Elle peut être fabriquée sous forme expansée, puis "écrasée" sur un ballonnet gonflable destiné à être monté à l'extrémité d'un cathéter.
Les barres formant les méandres auront avantageusement une section rectangulaire de largeur sensiblement égale à 0,12 mm et d'une épaisseur voisines de 0,1 mm, la longueur d'un module 2 étant de l'ordre de 4 mm.

Afin d'éviter que l'endothélium des vaisseaux de pénétrer à l'intérieur de la gaine expansible 1, à travers le grillage constituant sa paroi, celle-ci sera avantageusement recouverte d'un film souple en matériau synthétique tel que polyuréthanne, silicone ou polyester.
Pour faciliter les opérations de mise en place et éventuellement de retrait, la gaine 1 pourra comporter des repères radio-opaques visibles aux rayons X constitués de couches d'un métal lourd tel que le tantale, le titane ou l'or déposé par voie électrolytique.

Le positionnement des divers éléments constitutifs donne à l'objet de l'invention un maximum d'effets utiles qui n'avaient pas été, à ce jour, obtenus par des dispositifs similaires.

## Revendications

1. Gaine vasculaire interne expansible et cintrable à usage chirurgical, réalisée dans un matériau semi-rigide malléable lui permettant de s'étendre radialement sous l'effet d'une pression interne et de garder la forme ainsi obtenue, et destinée à la dilatation locale de conduits physiologiques tels que veines et artères dans le but de diminuer les troubles circulatoires et en particulier les risques d'infarctus provoqués par l'oblitération d'un vaisseau, par exemple du myocarde, constituée d'un ou plusieurs modules (2) cylindriques identiques de section circulaire formés chacun par l'assemblage de deux barres de section sensiblement constante décrivant des méandres périphériques et solidarisées de manière à former des motifs fermés se succédant le long de la périphérie de la gaine (1),
**caractérisée en ce que** chaque module (2) comprend quatre éléments longitudinaux (3) sinueux susceptibles d'être allongés ou raccourcis de manière à permettre le cintrage du module (2), ces éléments longitudinaux (3) étant formés d'une partie courbe (5) déformable insérée entre deux parties droites (6, 7) solidaires chacune d'un élément en arc de cercle (4) reliant deux éléments longitudinaux, les dits éléments en arcs de cercles étant alternés.

2. Gaine vasculaire interne selon la revendication 1, **se caractérisant par le fait qu'**un module (2) est formé de deux barres en méandres contiguës reliées par des barres courtes (8) solidaires de leurs éléments en arc de cercle (4) respectifs.

3. Gaine vasculaire interne selon l'une quelconque des revendications précédentes, **se caractérisant par le fait que** les barres formant les méandres ont une section rectangulaire de largeur sensiblement égale à 0,12 millimètre et d'une épaisseur voisine de 0,1 millimètre, la longueur d'un module (2) étant de l'ordre de 4 millimètres.

4. Gaine vasculaire interne selon l'une quelconque des revendications précédentes, **se caractérisant par le fait qu'**elle est constituée de plusieurs modules (2) reliés entre eux par une ou plusieurs barres de liaison courtes (10) disposées entre lesdits modules de façon contiguë, en quinconce ou décalées périphériquement d'un module à l'autre.

5. Gaine vasculaire interne selon l'une quelconque des revendications 1 à 3, **se caractérisant par le fait qu'**elle est constituée de plusieurs modules (2) reliés entre eux par une ou plusieurs barres de liaison longues (11), pouvant atteindre les deux tiers de la longueur d'un module, et disposées entre lesdits modules de façon contiguë, en quinconce ou décalées périphériquement d'un module à l'autre.

6. Gaine vasculaire interne selon l'une quelconque des revendications précédentes, **se caractérisant par le fait qu'**elle est réalisée en une seule pièce par gravure ou usinage laser d'un tube métallique à paroi mince.

7. Gaine vasculaire interne selon l'une quelconque des revendications 1 à 5, **se caractérisant par le fait qu'**elle est réalisée en une seule pièce par électro-érosion à partir d'un bloc métallique.

8. Gaine vasculaire interne selon l'une quelconque des revendications 6 et 7, **se caractérisant par le fait qu'**elle est réalisée en acier inoxydable ou en tantale.

9. Gaine vasculaire interne selon l'une quelconque des revendications précédentes, **se caractérisant par le fait que** sa paroi est recouverte d'un film souple en matériau synthétique tel que polyuréthanne, silicone ou polyester, de manière à empêcher l'endothélium du conduit traité de pénétrer à l'intérieur de la gaine à travers ladite paroi.

10. Gaine vasculaire interne expansible selon l'une quelconque des revendications précédentes, **se caractérisant par le fait qu'**elle est fabriquée sous forme expansée, puis "écrasée" sur un ballonnet gonflable destiné à être monté à l'extrémité d'un cathéter.

11. Gaine vasculaire interne selon l'une quelconque des revendications précédentes, **se caractérisant par le fait qu'**elle comporte des repères radio-opaques visibles aux rayons X constitués de couches d'un métal lourd déposé par voie électrolytique.

## Patentansprüche

1. Interne, erweiterbare und biegsame Gefäßstütze für chirurgische Zwecke aus einem formbaren, halbsteifen Material, das eine radiale Aufweitung unter Wirkung des Innendrucks und das Beibehalten der so erzielten Form erlaubt, bestimmt zur örtlichen Aufweitung physiologischer Gefäße, zum Beispiel Venen und Arterien, mit dem Ziel, Kreislaufbeschwerden, insbesondere das Risiko eines Herzinfarkts durch Verstopfung eines Gefäßes, beispielsweise des Herzmuskels zu verringern, bestehend aus einem oder mehreren gleichartigen, zylinderförmigen Modulen (2) mit kreisförmigem Querschnitt, die jeder aus zwei miteinander verbundenen Stäben mit annähernd konstantem Querschnitt bestehen, die auf dem Umfang mäanderförmig verlaufen und so miteinander verbunden sind, dass sie geschlossene geometrische Formen bilden, die sich auf dem Umfang der Gefäßstütze (1) in Längsrichtung aneinander reihen,
**gekennzeichnet dadurch, dass** jeder Modul (2) aus vier gewundenen Elementen (3) in Längsrichtung besteht, die verlängert oder verkürzt werden können, so das ein Biegen des Moduls (2) möglich ist, wobei die Elemente (3) in Längsrichtung aus einem gekrümmten, verformbaren Abschnitt (5) bestehen, der zwischen zwei gerade Abschnitte (6, 7) eingefügt wird, die miteinander durch einen Kreisbogen (4) verbunden sind, der wiederum zwei Längsrichtungs-Elemente miteinander verbindet, wobei die vorgenannten Kreisbogenelemente abwechselnd angeordnet sind.

2. Interne Gefäßstütze gemäß Patentanspruch 1, **gekennzeichnet dadurch, dass** ein Modul (2) aus zwei aneinandergereihten mäanderförmigen Stäben besteht, die miteinander durch kurze, mit den ihnen zugeordneten Kreisbogenelementen (4) verbunden Stäben (8) verbunden sind.

3. Interne Gefäßstütze gemäß einem der vorstehenden Patentansprüche, **gekennzeichnet dadurch, dass** die mäanderförmigen Stäbe einen rechteckigen Querschnitt haben, dessen Breite etwa 0,12 Millimeter und dessen Dicke näherungsweise 0,1 Millimeter betragen, wobei die Länge des Moduls (2) in der Größenordnung von 4 Millimeter liegt.

4. Interne Gefäßstütze gemäß einem der vorstehenden Patentansprüche, **gekennzeichnet dadurch, dass** sie aus mehreren Modulen (2) besteht, die miteinander durch einen oder mehrere kurze Verbindungsstäbe (10) verbunden sind, die zwischen den genannten Modulen auf dem Umfang aufeinanderfolgend, im Zickzack oder versetzt angeordnet sind.

5. Interne Gefäßstütze gemäß einem der Patentansprüche 1 bis 3, **gekennzeichnet dadurch, dass** sie aus mehreren Modulen (2) besteht, die miteinander durch einen oder mehrere lange Verbindungsstäbe (11) verbunden sind, die zwei Drittel der Länge eines Moduls erreichen können und zwischen den genannten Modulen auf dem Umfang aufeinanderfolgend, im Zickzack oder versetzt angeordnet sind.

6. Interne Gefäßstütze gemäß einem der vorstehenden Patentansprüche **gekennzeichnet dadurch, dass** sie durch Gravur oder Laserbearbeitung aus einem einzigen dünnwandigen Metallrohr hergestellt wurde.

7. Interne Gefäßstütze gemäß einem der Patentansprüche 1 bis 5, **gekennzeichnet dadurch, dass** sie durch Elektroerosion aus einem einzigen Metallblock hergestellt wurde.

8. Interne Gefäßstütze gemäß einem der Patentansprüche 1 bis 6, **gekennzeichnet dadurch, dass** sie aus Edelstahl oder Tantal hergestellt wurde.

9. Interne Gefäßstütze gemäß einem der vorstehenden Patentansprüche **gekennzeichnet dadurch, dass** ihre Oberfläche mit einer Kunststofffolie bedeckt ist, zum Beispiel mit Polyurethan, Silikon oder Polyester, so dass das Endothel des behandelten Gefäßes daran gehindert wird, durch die genannte Wandung ins Innere der Gefäßstütze einzudringen.

10. Interne erweiterbare Gefäßstütze gemäß einem der vorstehenden Patentansprüche, **gekennzeichnet dadurch, dass** sie in aufgeweiteter Form hergestellt wird und anschließend auf einem aufblasbaren Ballon "zusammengedrückt" wird, der zur Montage am Ende eines Katheters bestimmt ist.

11. Interne Gefäßstütze gemäß einem der vorstehenden Patentansprüche, **gekennzeichnet dadurch, dass** sie für Röntgenstrahlen opake Markierungen enthält, die im Röntgenbild sichtbar sind und aus einer Schwermetallschicht bestehen, die auf elektrolytischem Wege aufgebracht wird.

## Claims

1. An expandable and bendable internal vascular sheath for surgical use, made of a malleable semi-rigid material enabling it to expand radially under the effect of an internal pressure and to keep the shape thus obtained, and intended for the local dilation of physiological ducts such as veins and arteries with the aim of reducing circulatory disorders and in particular the risks of infarctus caused by the occlusion of a vessel, for instance the myocardium, consisting of one or more identical cylindrical modules (2) of circular section each formed by the assembly of two rods of roughly constant section describing peripheral meanders and joined together to form a succession of closed shapes along the periphery of the sheath (1),
**characterized in that** each module (2) comprises four sinuous longitudinal elements (3) that can be lengthened or shortened so as to allow the bending of the module (2), these longitudinal elements (3) being formed of a deformable curved part (5) inserted between two straight parts (6, 7) each joined to an arc-shaped element (4) connecting two longitudinal elements, the said arc-shaped elements being alternating.

2. An internal vascular sheath according to Claim 1, **characterized in that** a module (2) is formed of two rods in contiguous meanders connected by short rods (8) joined to their respective arc-shaped elements (4).

3. An internal vascular sheath according to any one of the preceding claims, **characterized in that** the rods forming the meanders have a rectangular section whose width is roughly equal to 0.12 millimeters and whose thickness is close to 0.1 millimeters, the length of a module (2) being of the order of 4 millimeters.

4. An internal vascular sheath according to any one of the preceding claims, **characterized in that** it consists of several modules (2) connected to each other by one or several short connecting rods (10) arranged between the said modules contiguously, staggered or offset peripherally from one module to another.

5. An internal vascular sheath according to any one of Claims 1 to 3, **characterized in that** it consists of several modules (2) connected to each other by one or several long connecting rods (11), up to two-thirds as long as a module, and arranged between the said modules contiguously, staggered or offset peripherally from one module to another.

6. An internal vascular sheath according to any one of the preceding claims, **characterized in that** it is made in a single piece by laser machining or etching of a thin-walled metal tube.

7. An internal vascular sheath according to any one of Claims I to 5, **characterized in that** it is made in a single piece by electroerosion from a metal block.

8. An internal vascular sheath according to any one of Claims 6 and 7, **characterized in that** it is made of stainless steel or of tantalum.

9. An internal vascular sheath according to any one of the preceding claims, **characterized in that** its wall is covered with a flexible film of synthetic material such as polyurethane, silicon or polyester, so as to prevent the endothelium of the treated duct from penetrating inside the sheath through the said wall.

10. An internal vascular sheath expandable according to any one of the preceding claims, **characterized in that** it is fabricated in expanded form, and then "crushed" on an inflatable balloon designed to be fitted on the end of a catheter.

11. An internal vascular sheath according to any one of the preceding claims, **characterized in that** it includes radio-opaque markings that are visible by X-ray formed of layers of a heavy metal deposited by electrolysis.
